# EUROPEAN PATENT APPLICATION

(11) **EP 4 545 136 A1**
(43) Date of publication of application: **30.04.2025**
(21) Application number: 22949010.7
(22) Date of filing: 13.10.2022
(51) Int. Cl.: A61N 2/04

(54) **MAGNETIC STIMULATION DEVICE AND MAGNETIC STIMULATION APPARATUS**

(30) Priority: 27.06.2022 CN 202210744897
(71) Applicant: Institute of Automation, Chinese Academy Sciences, Beijing 100190 (CN)
(72) Inventor: QI, Zihui, Beijing 100190 (CN); JIANG, Tianzai, Beijing 100190 (CN)
(74) Representative: Geskes, Christoph
(86) International application number: PCT/CN2022/125021
(87) International publication number: WO 2024/000925

(57) **Abstract**

Provided herein are a magnetic stimulation device and a magnetic stimulation apparatus. The present application relates to the technical field of biomedicine and solves the overweight problem of existing magnetic stimulation devices. The magnetic stimulation device comprises two first cylindrical magnetic cores arranged at an interval and coils. The coils wrap around the peripheries of the two first cylindrical magnetic cores, respectively. The coils are configured for generating a magnetic field when a current passes through and the first cylindrical magnetic cores are configured for enhancing the magnetic field, so as to stimulate a target subject through the enhanced magnetic field, thus achieving the purpose of reducing the weight of the magnetic stimulation device while providing sufficient magnetic stimulation intensity.

## Description

### CROSS-REFERENCE TO RELATED APPLICATION

The present application claims priority to Chinese Patent Application No. 202210744897.3, filed on June 27, 2022, entitled "Magnetic Stimulation Device and Magnetic Stimulation Apparatus", which is hereby incorporated by reference in its entirety.

### TECHNICAL FIELD

The present application relates to the field of biomedicine, and in particular to a magnetic stimulation device and a magnetic stimulation apparatus.

### BACKGROUND

Transcranial magnetic stimulation (TMS) is a stimulation technology that stimulates brain nerves with current induced in a cerebral cortex by intense alternating magnetic fields penetrating a skull.

In the related art, a magnetic stimulation device is usually used to perform transcranial magnetic stimulation and includes a "C"-shaped magnetic core and a coil wrapped around the "C"-shaped magnetic core. When the coil is energized, current passes through the coil to generate a magnetic field, and the "C"-shaped magnetic core may further strengthen the magnetic field, and transcranial magnetic stimulation is performed through the strengthened magnetic field.

There will be a problem that the magnetic core is easily saturated although the "C"-shaped magnetic core may greatly improve the intensity of magnetic stimulation. In order to avoid the saturation of the magnetic core, it is usually necessary to provide a cross-sectional area of the "C"-shaped magnetic core to be large enough, and a magnetic path of the "C"-shaped magnetic core to be long, but thus will result in a heavy magnetic stimulation device.

### SUMMARY

The present application provides a magnetic stimulation device and a magnetic stimulation device with a reduced weight while providing sufficient magnetic stimulation intensity.

The present application provides a magnetic stimulation device, including:
two first cylindrical magnetic cores and coils arranged at intervals respectively, where the coils are respectively wrapped around outer circumferences of the two first cylindrical magnetic cores.

The coils are used to generate a magnetic field when current passes through the coil.

The first cylindrical magnetic cores are used to strengthen the magnetic field to stimulate a target object through the strengthened magnetic field.

According to the magnetic stimulation device provided by the present application, the coils are wrapped around middle parts of the first cylindrical magnetic cores, and both ends of the first cylindrical magnetic cores are exposed outside the coil.

According to the magnetic stimulation device provided by the present application, a through hole is provided axially inside each of the first cylindrical magnetic cores.

According to the magnetic stimulation device provided by the present application, the magnetic stimulation device further includes second cylindrical magnetic cores provided at tops of the two first cylindrical magnetic cores, the first cylindrical magnetic cores and the second cylindrical magnetic cores are arranged coaxially, and a diameter of the second cylindrical magnetic cores is greater than a diameter of the first cylindrical magnetic cores.

According to the magnetic stimulation device provided by the present application, through holes are provided axially inside both the first cylindrical magnetic cores and the second cylindrical magnetic cores and top ends of the first cylindrical magnetic cores are provided inside the through holes of the second cylindrical magnetic cores.

According to the magnetic stimulation device provided by the present application, recesses are provided at sides of the second cylindrical magnetic cores away from the first cylindrical magnetic cores, and protrusions are formed at sides of the first cylindrical magnetic cores away from the second cylindrical magnetic cores.

According to the magnetic stimulation device provided by the present application, axes of the two first cylindrical magnetic cores form a predetermined angle, sides of the two first cylindrical magnetic cores facing the target object are tangent to a stimulated site of the target object, and the second cylindrical magnetic cores are located at sides of the first cylindrical magnetic cores away from the stimulated site.

According to the magnetic stimulation device provided by the present application, the coils are wrapped in a single layer in axial directions of the two first cylindrical magnetic cores and wrapped in multiple layers in the radial directions of the two first cylindrical magnetic cores, and two ends of the coils are respectively led out from inner rings of circular coils close to the first cylindrical magnetic cores.

Current directions in the two circular coils wrapped around the two first cylindrical magnetic cores are opposite.

According to the magnetic stimulation device provided by the present application, the coils are wrapped in multiple layers in axial directions of the two first cylindrical magnetic cores and wrapped in multiple layers in the radial direction of the two first cylindrical magnetic cores, two ends of the coils are respectively led out from inner rings of circular coils away from the first cylindrical magnetic cores when a number of layers wrapped in the axial direction is an odd number; two ends of the coil are respectively led out from outer rings of circular coils away from the first cylindrical magnetic core when a number of layers wrapped in the axial direction is an even number.

Current directions in the two circular coils wrapped around the two first cylindrical magnetic cores are opposite.

The present application further provides a magnetic stimulation apparatus, including: a housing, and a magnetic stimulation device provided inside the housing; where the magnetic stimulation device is any one of the magnetic stimulation devices described above.

In the magnetic stimulation device and magnetic stimulation apparatus provided by the present application, the magnetic stimulation device includes two first cylindrical magnetic cores and coils arranged at intervals respectively, and the coils are respectively wrapped around outer circumferences of the two first cylindrical magnetic cores. The coils are used to generate a magnetic field when current passes through the coils; and the first cylindrical magnetic cores are used to strengthen the magnetic field to stimulate the target object through the strengthened magnetic field. Through the cylindrical magnetic cores arranged in the center of the coil, a stimulation intensity required for magnetic stimulation may be effectively provided, and the magnetic path of the first cylindrical magnetic core is shorter, thereby reducing the weight of the magnetic stimulation device while providing sufficient magnetic stimulation intensity.

### BRIEF DESCRIPTION OF THE DRAWINGS

To illustrate solutions of the present application or in the related art more clearly, accompanying drawings used in the description of the embodiments or the related art are briefly described below. The drawings described below are only some embodiments of the present application. For those skilled in the art, other drawings may also be obtained based on these drawings without creative effort.
FIG. 1 is a schematic diagram of an application scenario according to the present application;
FIG. 2 is a schematic diagram of magnetic field distribution of a coil according to the present application;
FIG. 3 is a schematic structural diagram in front view of a magnetic stimulation device according to the present application;
FIG. 4 is a schematic structural diagram in top view of a magnetic stimulation device according to the present application;
FIG. 5 is a schematic structural diagram in which a coil is wrapped around middle parts and lower ends of the first cylindrical magnetic cores, and upper ends of the first cylindrical magnetic cores are exposed outside the coils according to the present application;
FIG. 6 is a first schematic diagram of a magnetic stimulation intensity according to the present application;
FIG. 7 is a schematic structural diagram in front view of a magnetic stimulation device including first cylindrical magnetic cores and second cylindrical magnetic cores according to the present application;
FIG. 8 is a schematic structural diagram in top view of a magnetic stimulation device including first cylindrical magnetic cores and second cylindrical magnetic cores according to the present application;
FIG. 9 is a schematic structural diagram in bottom view of a magnetic stimulation device including first cylindrical magnetic cores and second cylindrical magnetic cores according to the present application;
FIG. 10 is a second schematic diagram of a magnetic stimulation intensity according to the present application;
FIG. 11 is a schematic structural diagram in top view of a magnetic stimulation device including first cylindrical magnetic cores and second cylindrical magnetic cores provided with through holes according to the present application;
FIG. 12 is a schematic structural diagram in bottom view of a magnetic stimulation device including first cylindrical magnetic cores and second cylindrical magnetic cores provided with through holes according to the present application;
FIG. 13 is a schematic structural diagram in front view of a magnetic stimulation device provided with recesses and protrusions according to the present application;
FIG. 14 is a schematic diagram of a relationship between a magnetic stimulation device and a stimulated site according to the present application; and
FIG. 15 is a third schematic diagram of a magnetic stimulation intensity according to the present application.

### Reference numerals:

30: magnetic stimulation device; 301: first cylindrical magnetic core; 302: coil; 303: second cylindrical magnetic core.

### DETAILED DESCRIPTION

To illustrate objectives, solutions and advantages of the present application, the solutions of the present application are described clearly and completely below in combination with the drawings in the present application. The described embodiments are part of the embodiments of the present application, not all of the embodiments. All other embodiments obtained by those skilled in the art based on the embodiments of the present application without any creative effort shall fall within the scope of the present application.

In embodiments of the present application, "at least one" means one or more, and "multiple" means two or more. "And/or" describes an association relationship of associated objects, indicating that there may be three relationships. For example, A and/or B may represent that merely A exists, both A and B exist, and merely B exists. A and B can be singular or plural. In text description of the present application, character "/" generally indicates that the associated objects before and after are in an "or" relationship.

Transcranial magnetic stimulation (TMS) is a stimulation technology that stimulates brain nerves with current induced in a cerebral cortex by intense alternating magnetic fields penetrating a skull. TMS can not only used for transcranial nerve regulation, but also for peripheral nerve and muscle stimulation. TMS has been applied in various fields such as neuropsychology, rehabilitation, and pediatrics.

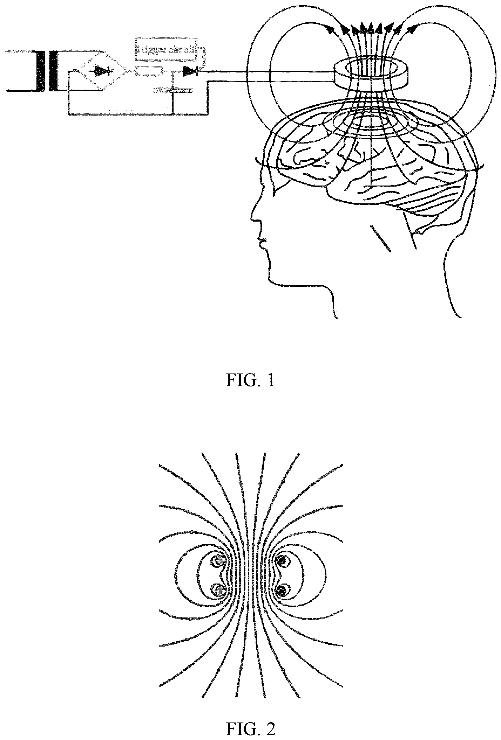

For example, as shown in FIG. 1, which is a schematic diagram of an application scenario according to the present application, the present application provides a magnetic stimulation device and a pulse power supply for energizing the magnetic stimulation device in a transcranial magnetic stimulation scenario. The magnetic stimulation device shown in FIG. 1 only includes a coil. When the magnetic stimulation device is energized, current passes through the coil to generate a magnetic field, thereby performing transcranial magnetic stimulation through the magnetic field.

However, since the instantaneous current passing through the coil reaches more than thousands of amperes, a pulse width of a single pulse is about 350 us, a repetitive stimulation frequency may reach 100 Hz, and energy consumed by a single stimulation pulse ranges from tens of joules to hundreds of joules, thus energy ultimately consumed in the transcranial magnetic stimulated site is relatively small, and most of the energy is dissipated in the coil, a wire, and a circuit in the form of heat; air cooling and water cooling are required to dissipate the heat of the coil during the magnetic stimulation process.

To solve the problems in the above-mentioned magnetic stimulation device that only includes a coil, a magnetic stimulation device including a "C"-shaped magnetic core and a coil wrapped around the "C"-shaped magnetic core is usually used for transcranial magnetic stimulation in the related art. There will be a problem that the magnetic core is easily saturated although the "C"-shaped magnetic core may greatly improve the intensity of magnetic stimulation. In order to avoid the saturation of the magnetic core, it is usually necessary to provide a cross-sectional area of the "C"-shaped magnetic core to be large enough, and a magnetic path of the "C"-shaped magnetic core to be long, but thus will result in a heavy magnetic stimulation device.

In order to reduce the weight of the magnetic stimulation device while providing sufficient magnetic stimulation intensity, the magnetic field distribution of the coil is observed. For example, as shown in FIG. 2, which is a schematic diagram of magnetic field distribution of a coil according to the present application, it can be seen in conjunction with FIG. 2, the densest part in the magnetic field is an inner diameter part of the coil, and a magnetic resistance of this inner diameter part of the coil is the largest. The magnetic field space is large and the equivalent magnetic resistance is small at both sides of the coil. Therefore, if a cylindrical magnetic core is provided at the center of the coil, a magnetic stimulation intensity may be greatly improved, and the weight of the magnetic stimulation device may be reduced compared with the "C" type magnetic core.

Based on the above concept, the present application provides a magnetic stimulation device. The magnetic stimulation device according to the present application will be described in detail below through specific embodiments. It may be understood that the following specific embodiments may be combined with each other, and the same or similar concepts or procedures may not be repeated in some embodiments.

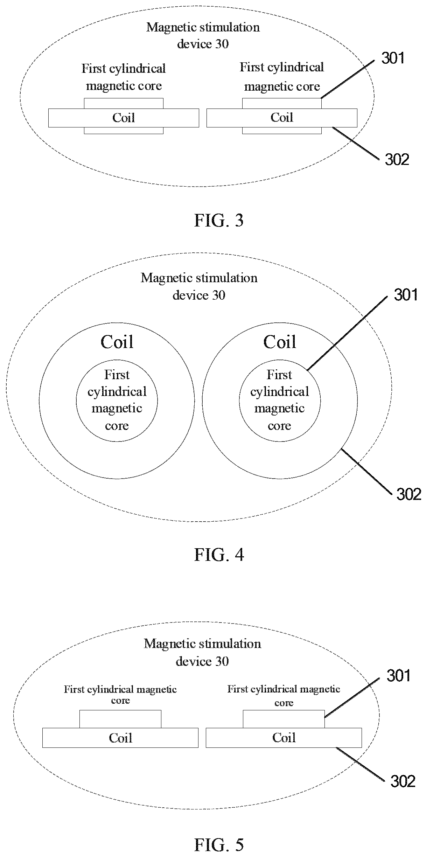

FIG. 3 is a schematic structural diagram in front view of a magnetic stimulation device 30 according to the present application. For example, as shown in FIG. 3, the magnetic stimulation device 30 may include:
two first cylindrical magnetic cores 301 and coils 302 arranged at intervals respectively, where the coils 302 are respectively wrapped around outer circumferences of the two first cylindrical magnetic cores 301.

The coils 302 are used to generate a magnetic field when current passes through the coils.

The first cylindrical magnetic cores 301 are used to strengthen the magnetic field to stimulate a target object through the strengthened magnetic field.

For ease of understanding, in combination with FIG. 4, FIG. 4 is a schematic structural diagram in top view of a magnetic stimulation device 30 according to the present application. The magnetic stimulation device 30 includes two first cylindrical magnetic cores 301 and coils 302 arranged at intervals respectively, and the coils 302 are respectively wrapped around outer circumferences of the two first cylindrical magnetic cores 301.

As an example, a height of the first cylindrical magnetic core 301 may be any value ranging from 0.5 cm to 5 cm, and a diameter of the first cylindrical magnetic core 301 may be any value ranging from 4 cm to 7 cm, which may be set according to actual needs.

As an example of the first cylindrical magnetic cores 301 with a height of 1 cm and a diameter of 5 cm, a magnetic field generated by the coils 302 is strengthened by the first cylindrical magnetic cores 301. It may be concluded from software simulation experiments that: compared with the 0.27 T of magnetic stimulation intensity provided by the coils 302 alone, the magnetic stimulation intensity about 2 cm below the coil 302 may be increased to 0.33 T, and the weight of the first cylindrical magnetic cores 301 is only 300 g, which effectively reduces the weight of the magnetic stimulation device 30 compared with the "C" type magnetic core.

As an example of the first cylindrical magnetic cores 301 with a height of 2 cm and a diameter of 5 cm, a magnetic field generated by the coils 302 is strengthened by the first cylindrical magnetic cores 301. It may be concluded from software simulation experiments that: compared with the 0.27 T of magnetic stimulation intensity provided by the coils 302 alone, the magnetic stimulation intensity about 2 cm below the coils 302 may be increased to 0.39 T, and the weight of the first cylindrical magnetic core 301 is only 600g, which effectively reduces the weight of the magnetic stimulation device 30 compared with the "C" type magnetic core.

In combination with two examples above, it is not difficult to see that the greater the height of the first cylindrical magnetic cores 301, the stronger the corresponding magnetic stimulation intensity provided, that is, the height of the first cylindrical magnetic cores 301 is directly proportional to the magnetic stimulation intensity it provides. However, the height of the first cylindrical magnetic cores 301 should not be too high, because if the height is too high, the weight of the magnetic stimulation device 30 will increase, thereby failing to reduce weight.

As an example, in an embodiment of the present application, the first cylindrical magnetic cores 301 may be formed by pressing highly saturated magnetic powder material through a customized mold, and it has good magnetic permeability and magnetic saturation performance. The first cylindrical magnetic cores 301 may also be formed by mixing adhesives such as epoxy resin, high-temperature paraffin wax with magnetic powder and performing injection molding. The first cylindrical magnetic cores 301 may also be formed by combing and fixing standard toroidal magnetic cores and cylindrical magnetic cores that are commercially available through socketing. Epoxy resin may be used for fixing, and magnetic powder may be added to the epoxy resin to improve the magnetic permeability at the gap, which may be set according to actual needs.

It can be seen that the magnetic stimulation device 30 provided by the present application includes two first cylindrical magnetic cores 301 and coils 302 arranged at intervals respectively, and the coils 302 are respectively wrapped around outer circumferences of the two first cylindrical magnetic cores 301. The coils 302 are used to generate a magnetic field when current passes through the coils; the first cylindrical magnetic cores 301 are used to strengthen the magnetic field to stimulate the target object through the strengthened magnetic field. Through the cylindrical magnetic cores arranged in the center of the coils 302, a stimulation intensity required for magnetic stimulation may be effectively provided, thereby reducing the weight of the magnetic stimulation device 30 while providing sufficient magnetic stimulation intensity.

Based on the embodiment shown in FIG. 3, when the coils 302 in the magnetic stimulation device 30 is respectively wrapped around outer circumferences of the two first cylindrical magnetic cores 301, outer circumferences of the first cylindrical magnetic cores 301 may be completely wrapped or partially wrapped, which may be set according to actual needs.

In a possible scenario, in order to reduce the ineffective magnetic field energy at upper ends of the first cylindrical magnetic cores 301 and guide the magnetic field closer to the magnetic stimulated site, bas an example, in the embodiment of the present application, the coils 302 may be wrapped around middle parts of the first cylindrical magnetic cores 301, and both ends of the first cylindrical magnetic cores 301 are exposed outside the coils 302, as shown in FIG. 3. By exposing both ends of the first cylindrical magnetic cores 301 outside the coils 302, the ineffective magnetic field energy at the upper end of the first cylindrical magnetic core 301 may be reduced, and the magnetic field may be guided closer to the magnetic stimulated site.

In this possible scenario, when the coils 302 are wrapped around middle parts of the first cylindrical magnetic cores 301 and both ends of the first cylindrical magnetic cores 301 are exposed outside the coils 302, the greater the height of the first cylindrical magnetic cores 301, the stronger the corresponding magnetic stimulation intensity provided.

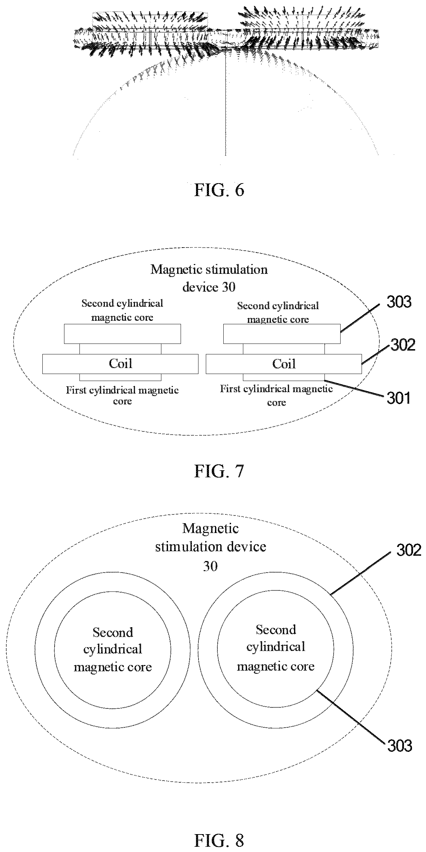

When the coils 302 are respectively wrapped around outer circumferences of the two first cylindrical magnetic cores 301, the coils 302 may also be wrapped around a middle part and one end part of the first cylindrical magnetic cores 301, and the other end part of the first cylindrical magnetic cores 301 is exposed outside the coils 302. For example, the coils 302 may be wrapped around a middle part and an upper end of the first cylindrical magnetic cores 301, and lower ends of the first cylindrical magnetic cores 301 are exposed outside the coils 302, and the magnetic field may be guided closer to the magnetic stimulated site. Alternatively, the coils 302 may be wrapped around middle parts and lower ends of the first cylindrical magnetic cores 301, and upper ends of the first cylindrical magnetic cores 301 are exposed outside the coils 302. As an example, as shown in FIG. 5 which is a schematic structural diagram, coils 302 are wrapped around middle parts and lower ends of the first cylindrical magnetic core 301, and upper ends of the first cylindrical magnetic cores are exposed outside the coils 302 according to the present application, this may reduce the ineffective magnetic field energy at upper ends of the first cylindrical magnetic cores 301. Accordingly, the magnetic stimulation device 30 shown in FIG. 5 may refer to FIG. 6, which is a first schematic diagram of a magnetic stimulation intensity according to the present application.

In a possible scenario, based on the embodiment shown in FIG. 3, on the basis of the magnetic stimulation device 30 provided in the embodiment shown in FIG. 3, in order to further reduce the weight of the magnetic stimulation device 30, it is found by observing a distribution of magnetic induction intensity that the magnetic induction intensity of the central part of the first cylindrical magnetic cores 301 is relatively weak. Material in the center of the first cylindrical magnetic cores 301 may be removed under the premise of not causing core saturation, a through hole is provided axially inside each of the first cylindrical magnetic cores 301 to form a toroidal-cylindrical magnetic core, thereby achieving the purpose of reducing the weight of the magnetic stimulation device 30 through the toroidal-cylindrical magnetic core provided with a through hole.

As an example, when through holes are provided axially inside the first cylindrical magnetic cores 301, a diameter of the through holes may be any value ranging from 1 cm to 2 cm, as long as the diameter of the through holes is smaller than the diameter of the first cylindrical magnetic cores 301, and it may be set according to actual needs.

In a possible scenario, based on the embodiment shown in FIG. 3, on the basis of the magnetic stimulation device 30 provided in the embodiment shown in FIG. 3, in order to further improve the stimulation intensity of the magnetic stimulation device 30 and reduce the magnetic resistance, second cylindrical magnetic cores 303 is provided at tops of the two first cylindrical magnetic cores 301. That is, second cylindrical magnetic cores are provided at tops of first cylindrical magnetic cores, the first cylindrical magnetic cores 301 and the second cylindrical magnetic cores 303 are arranged coaxially, and a diameter of the second cylindrical magnetic cores 303 is greater than a diameter of the first cylindrical magnetic cores 301. For example, as shown in FIG. 7, Fig. 7 is a schematic structural diagram in front view of a magnetic stimulation device including first cylindrical magnetic cores 301 and second cylindrical magnetic cores 303 according to the present application. It can be seen in conjunction with FIG. 7, the first cylindrical magnetic cores 301 and the second cylindrical magnetic cores 303 constitute a "T"-shaped double cylindrical magnetic core. It may be understood that no coil 302 is wrapped around the second cylindrical magnetic cores 303.

As an example, a height of the second cylindrical magnetic cores 303 may be any value ranging from 0.5 cm to 3 cm, and a diameter of the second cylindrical magnetic cores 303 may be any value ranging from 2 cm to 5 cm, and the diameter of the second cylindrical magnetic cores 303 is greater than the diameter of the first cylindrical magnetic cores 301, which may be set according to actual needs.

It should be noted that in the embodiment of the present application, the "T"-shaped double cylindrical magnetic core consisting of the first cylindrical magnetic cores 301 and the second cylindrical magnetic cores 303 may be two cylindrical magnetic cores produced separately, and a "T"-shaped double cylindrical magnetic core can be obtained by combining them; it may also be a "T"-shaped double cylindrical magnetic core produced by integrated molding, which may be set according to actual needs, and does not limit thereto herein in the embodiment of the present application.

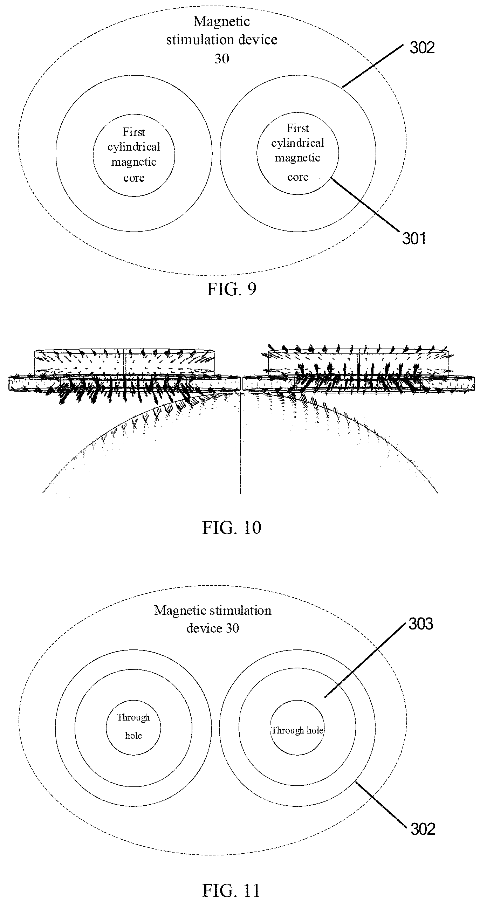

For ease of understanding, as shown in FIG. 8 and FIG. 9, FIG. 8 is a schematic structural diagram in top view of a magnetic stimulation device including first cylindrical magnetic cores 301 and second cylindrical magnetic cores 303 according to the present application, and FIG. 9 is a schematic structural diagram in bottom view of a magnetic stimulation device including first cylindrical magnetic cores 301 and second cylindrical magnetic cores 303 according to the present application. It may be seen in conjunction with FIG. 8 and FIG. 9 that the second cylindrical magnetic core 303 is provided at tops of the first cylindrical magnetic cores 301 in the magnetic stimulation device 30, and the first cylindrical magnetic cores 301 and the second cylindrical magnetic cores 303 are arranged coaxially, and a diameter of the second cylindrical magnetic cores 303 is greater than a diameter of the first cylindrical magnetic cores 301.

In conjunction with the magnetic stimulation device 30 shown in FIG. 7, by providing the second cylindrical magnetic cores 303 at the tops of the two first cylindrical magnetic cores 301, the magnetic stimulation intensity provided by the corresponding magnetic stimulation device 30 may be increased from 0.27 T (hollow) to 0.46 T at about 2cm below the coils 302, compared with the magnetic stimulation intensity provided by the coils 302. Increase in the weight of the magnetic stimulation device 30 is within an acceptable range since the volume of the magnetic core does not be increased much.

As an example, in this possible scenario, when the coils 302 in the "T"-shaped double cylindrical magnetic core is only wrapped around middle parts and lower ends of the first cylindrical magnetic cores 301, and the upper end is exposed outside the coils 302, the corresponding magnetic stimulation intensity may be seen in FIG. 10, which is a second schematic diagram of a magnetic stimulation intensity according to the present application. Compared with the magnetic stimulation intensity shown in FIG. 6, its magnetic stimulation intensity is significantly strengthened.

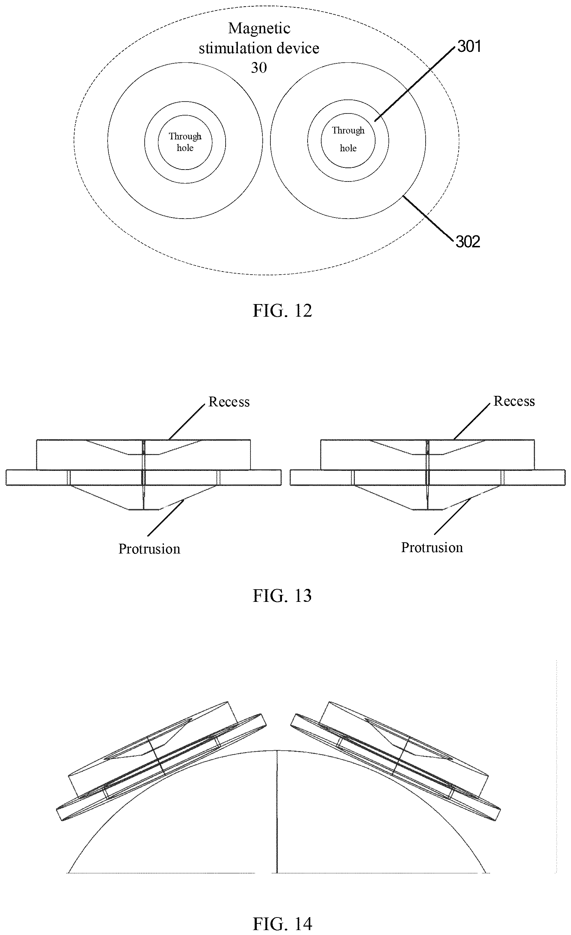

In a possible scenario, as an example, in conjunction with the magnetic stimulation device 30 shown in FIG. 7, in order to further reduce the weight of the magnetic stimulation device 30, it is found by observing a distribution of magnetic induction intensity that the magnetic induction intensity of central parts of the first cylindrical magnetic cores 301 is relatively weak. Material in the center of the first cylindrical magnetic cores 301 and the second cylindrical magnetic cores 303 may be removed under the premise of not causing core saturation, through holes are provided axially inside the first cylindrical magnetic cores 301 and the second cylindrical magnetic cores 303 to form a toroidal-cylindrical magnetic core, and the top ends of the first cylindrical magnetic cores 301 are provided inside through holes of the second cylindrical magnetic cores 303.As an example, as shown in FIG. 11 and FIG. 12, FIG. 11 is a schematic structural diagram in top view of a magnetic stimulation device including first cylindrical magnetic cores 301 and second cylindrical magnetic cores 303 provided with through holes according to the present application and FIG. 12 is a schematic structural diagram in bottom view of a magnetic stimulation device including first cylindrical magnetic cores 301 and second cylindrical magnetic cores 303 provided with through holes according to the present application. It may be seen in conjunction with FIG. 11 and FIG. 12 that through holes are provided axially inside both the first cylindrical magnetic cores 301 and the second cylindrical magnetic cores 303, and top ends of the first cylindrical magnetic cores 301 are provided inside the through holes of the second cylindrical magnetic cores 303. The weight of the magnetic stimulation device 30 may be effectively reduced by providing the toroidal-cylindrical magnetic core with through holes.

In this possible scenario, as an example, a diameter of through holes of the second cylindrical magnetic cores 303 may be approximately equal to the diameter of the first cylindrical magnetic cores 301, and top ends of the first cylindrical magnetic cores 301 may be provided inside the through holes of the second cylindrical magnetic cores 303. As an example, the diameter of the through holes of the first cylindrical magnetic cores 301 may be any value ranging from 1 cm to 2 cm, as long as the diameter of the through holes of the first cylindrical magnetic cores 301 is smaller than the diameter of the first cylindrical magnetic cores 301. The diameter of the through holes provided in the second cylindrical magnetic cores 303 may be determined based on the diameter of the through holes of the first cylindrical magnetic cores 301, as long as the diameter of the through holes of the second cylindrical magnetic cores 303 is smaller than the diameter of the second cylindrical magnetic cores 303 and may be set according to actual needs.

In a possible scenario, as an example, in conjunction with the magnetic stimulation device 30 shown in FIG. 7, considering that the magnetic induction intensity of central parts of the second cylindrical magnetic cores 303 away from the first cylindrical magnetic cores 301 is weak, the role of the magnetic core cannot be fully played, therefore, in order to further reduce the weight, the central part of the second cylindrical magnetic core may be removed, and recesses, which may be cone-shaped recesses, is provided at sides of the second cylindrical magnetic cores 303 away from the first cylindrical magnetic cores 301. In addition, in order to guide the magnetic field close to a stimulated site to improve the stimulation intensity, the removed cone may be moved to sides of the first cylindrical magnetic cores 301 away from the second cylindrical magnetic cores 303, and protrusions, which is coupled with the recesses, are formed at sides of the first cylindrical magnetic cores 301 away from the second cylindrical magnetic cores 303. As an example, as shown in FIG. 13, FIG. 13 is a schematic structural diagram in front view of a magnetic stimulation device 30 provided with recesses and protrusions according to the present application. It may be seen in conjunction with FIG. 13 that recesses are provided at sides of the second cylindrical magnetic cores 303 away from the first cylindrical magnetic cores 301, and protrusions are formed at sides of the first cylindrical magnetic cores 301 away from the second cylindrical magnetic cores 303.

It is assumed that the magnetic stimulation intensity at about 2 cm below the coils 302 of the magnetic stimulation device 30 shown in FIG. 7 is 0.37 T, by providing the recesses at sides of the second cylindrical magnetic cores 303 and the protrusions at sides of the first cylindrical magnetic cores 301, for example, the recesses and the protrusions may be coupled with each other. Referring to the magnetic stimulation device 30 shown in FIG. 13, the magnetic stimulation intensity at about 2 cm below the coil 302 is 0.45 T.

In a possible scenario, as an example, in conjunction with the magnetic stimulation device 30 shown in FIG. 7, when magnetic stimulation is performed on the stimulated site, since the magnetic stimulated site is arc-shaped, and the axes of the two first cylindrical magnetic cores 301 are on the same horizontal line, it will not only make the volume of the magnetic stimulation device 30 larger, but also fail to fit well with the stimulated site, thereby affecting the intensity and depth of the magnetic stimulation. In order to reduce the volume of the magnetic stimulation device 30 and make the magnetic stimulation device 30 fit well with the stimulated site, the two first cylindrical magnetic cores 301 may be bent inwardly, and the axes of the two first cylindrical magnetic cores 301 may form a predetermined angle, sides of the two first cylindrical magnetic cores 301 facing the target object are tangent to the stimulated site of the target object, and the second cylindrical magnetic cores 303 are located at sides of the first cylindrical magnetic cores 301 away from the stimulated site, thereby forming a double cone magnetic core. As an example, as shown in FIG. 14, FIG. 14 is a schematic diagram of a relationship between a magnetic stimulation device 30 and a stimulated site according to the present application. Although some focusing is lost, the height of the magnetic stimulation device 30 may be reduced, and the magnetic stimulation device 30 may be well fitted with the stimulated site, thereby effectively improving the intensity and depth of magnetic stimulation.

A value of the predetermined angle may be set according to actual needs. Here, the embodiments of the present application do not limit the value of the predetermined angle.

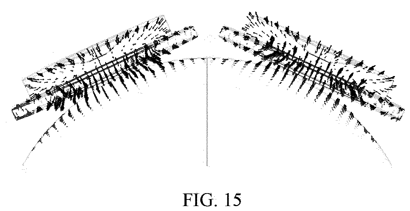

As an example, in this possible scenario, when the coils 302 in the magnetic stimulation device 30 shown in FIG. 14 are only wrapped around a middle part and a lower end of the first cylindrical magnetic core 301, and the upper end is exposed outside the coils 302, the corresponding magnetic stimulation intensity may be seen in FIG. 15. FIG. 15 is a third schematic diagram of a magnetic stimulation intensity according to the present application. Compared with the magnetic stimulation intensity shown in FIG. 10, its magnetic stimulation intensity is significantly strengthened.

Based on any of the above possible scenarios, for the magnetic stimulation device 30 shown in any possible scenario, when the coils 302 are respectively wrapped around outer circumferences of the two first cylindrical magnetic cores 301, it may include at least two possible wrapping methods as follows, in which the two first cylindrical magnetic cores 301 share one coil 302.

In one possible wrapping method, a disc-shaped figure-of-eight coil 302 in a single layer may be used to wrap around outer circumferences of the two first cylindrical magnetic cores 301. Specifically, the coils 302 are wrapped in a single layer in the axial directions of the two first cylindrical magnetic cores 301 and wrapped in multiple layers in the radial directions of the two first cylindrical magnetic cores 301, and two ends of the coil 302 are respectively led out from inner rings of circular coils 302 close to the first cylindrical magnetic cores 301. Current directions in the two circular coils 302 wrapped around the two first cylindrical magnetic cores 301 are opposite.

In this possible wrapping method, each circular coil 302 of the figure-of-eight coil 302 includes a layer of disc-shaped coil 302, and the coil 302 may be wound with flat copper wire or Litz wire. Litz wire is lighter and more conducive to reducing the weight of the magnetic stimulation device 30 under the same alternating current resistance. In addition, in order to reduce the resistance loss of the coils 302, it is necessary to increase the cross-sectional area of the wire as much as possible to reduce the alternating current resistance, a wider and thinner flat copper wire or rectangular Litz wire may be used, and a wider and thinner flat copper wire or rectangular Litz wire may be used to implement multi-layer winding in the radial direction of the two first cylindrical magnetic cores 301.

In another possible wrapping method, a disc-shaped figure-of-eight coil 302 in double layers may be used to wrap around outer circumferences of the two first cylindrical magnetic cores 301. Specifically, the coils 302 are wrapped in multiple layers in axial directions of the two first cylindrical magnetic cores 301 and wrapped in multiple layers in the radial directions of the two first cylindrical magnetic cores 301, two ends of the coil are respectively led out from inner rings of circular coils away from the first cylindrical magnetic cores 301 when a number of layers wrapped in the axial direction is an odd number; two ends of the coil are respectively led out from outer rings of circular coils away from the first cylindrical magnetic cores 301 when a number of layers wrapped in the axial direction is an even number. Current directions in the two circular coils wrapped around the two first cylindrical magnetic cores 301 are opposite.

In this possible wrapping method, each circular coil 302 of the figure-of-eight coils 302 includes at least two layers of disc-shaped coils 302, which communicate at the inner ring, and the two circular coils 302 communicate at the tangent of the outer ring, thereby forming a disc-shaped figure-of-eight coil 302 in double layers. Compared with the disc-shaped figure-of-eight coil302 in single layer, the double-layer coil 302 has fewer turns per layer. Thicker wires and low-cost round Litz wires may be used to effectively reduce the loss of the coil 302. Simultaneously, the lead wires of the coils 302 are all led out from the outer ring of the coils 302, which not only does not increase the thickness of the coils 302, but also makes wiring more convenient.

The present application further provides a magnetic stimulation apparatus. As an example, it may include: a housing, and a magnetic stimulation device provided inside the housing. The magnetic stimulation device is the magnetic stimulation devices shown in any of the above embodiments, and its implementation principle and beneficial effects of the magnetic stimulation device are similar to the implementation principle and beneficial effects of the magnetic stimulation device. Please refer to the implementation principle and beneficial effects of the magnetic stimulation device, and they will not be repeated here.

It should be noted that the above embodiments are only used to explain the solutions of the present application, and are not limited thereto; although the present application has been described in detail with reference to the foregoing embodiments, it should be understood by those skilled in the art that modifications to the solutions documented in the foregoing embodiments and equivalent substitutions to a part of the features may be made. However, these modifications and substitutions do not make the essence of the corresponding solutions depart from the scope of the solutions of various embodiments of the present application.

## Claims

1. A magnetic stimulation device, comprising:
two first cylindrical magnetic cores and coils arranged at intervals respectively, wherein the coils are respectively wrapped around outer circumferences of the two first cylindrical magnetic cores;
wherein the coils are used to generate a magnetic field when current passes through the coil; and
the first cylindrical magnetic cores are used to strengthen the magnetic field to stimulate a target object through the strengthened magnetic field.

2. The magnetic stimulation device of claim 1, wherein the coils are wrapped around middle parts of the first cylindrical magnetic cores, and both ends of the first cylindrical magnetic cores are exposed outside the coil.

3. The magnetic stimulation device of claim 1 or 2, wherein a through hole is provided axially inside each of the first cylindrical magnetic cores.

4. The magnetic stimulation device of claim 1 or 2, wherein the magnetic stimulation device further comprises second cylindrical magnetic cores provided at tops of the two first cylindrical magnetic cores, the first cylindrical magnetic cores and the second cylindrical magnetic cores are arranged coaxially, and a diameter of the second cylindrical magnetic cores is greater than a diameter of the first cylindrical magnetic cores.

5. The magnetic stimulation device of claim 4, wherein through holes are provided axially inside both the first cylindrical magnetic cores and the second cylindrical magnetic cores, and top ends of the first cylindrical magnetic cores are provided inside through holes of the second cylindrical magnetic cores.

6. The magnetic stimulation device of claim 4, wherein recesses are provided at sides of the second cylindrical magnetic cores away from the first cylindrical magnetic cores, and protrusions are formed at sides of the first cylindrical magnetic cores away from the second cylindrical magnetic cores.

7. The magnetic stimulation device of claim 4, wherein axes of the two first cylindrical magnetic cores form a predetermined angle, sides of the two first cylindrical magnetic cores facing the target object are tangent to a stimulated site of the target object, and the second cylindrical magnetic cores are located at sides of the first cylindrical magnetic cores away from the stimulated site.

8. The magnetic stimulation device of claim 1 or 2, wherein the coils are wrapped in a single layer in axial directions of the two first cylindrical magnetic cores and wrapped in multiple layers in the radial directions of the two first cylindrical magnetic cores, and two ends of the coils are respectively led out from inner rings of circular coils close to the first cylindrical magnetic cores; and
current directions in the two circular coils wrapped around the two first cylindrical magnetic cores are opposite.

9. The magnetic stimulation device of claim 1 or 2, wherein the coils are wrapped in multiple layers in axial directions of the two first cylindrical magnetic cores and wrapped in multiple layers in the radial directions of the two first cylindrical magnetic cores, two ends of the coils are respectively led out from inner rings of circular coils away from the first cylindrical magnetic cores when a number of layers wrapped in the axial direction is an odd number; two ends of the coils are respectively led out from outer rings of circular coils away from the first cylindrical magnetic cores when a number of layers wrapped in the axial direction is an even number; and
current directions in the two circular coils wrapped around the two first cylindrical magnetic cores are opposite.

10. A magnetic stimulation apparatus, comprising: a housing, and a magnetic stimulation device provided inside the housing; wherein the magnetic stimulation device is the magnetic stimulation devices of any one of claims 1 to 9.
